(19) Europäisches Patentamt
European Patent Office
Office européen des brevets

(11) **EP 3 401 681 B1**

(12) **EUROPEAN PATENT SPECIFICATION**

(45) Date of publication and mention of the grant of the patent:
**29.03.2023 Bulletin 2023/13**

(21) Application number: **18168500.9**

(22) Date of filing: **20.04.2018**

(51) International Patent Classification (IPC):
**G01N 33/558** (2006.01)   **G01N 33/68** (2006.01)
**G01N 33/82** (2006.01)

(52) Cooperative Patent Classification (CPC):
**G01N 33/82; G01N 33/558; G01N 33/6854**

(54) **METHOD FOR QUICK QUANTIFICATION ASSAY OF VITAMIN D BASED ON FLUORESCENCE MEASUREMENT USING LATERAL FLOW CARTRIDGE**

VERFAHREN ZUR SCHNELLEN QUANTITATIVEN BESTIMMUNG VON VITAMIN D AUF DER BASIS EINER FLUORESZENZMESSUNG UNTER VERWENDUNG EINER LATERALEN STRÖMUNGSKARTUSCHE

PROCÉDÉ DE DOSAGE QUANTITATIF RAPIDE DE LA VITAMINE D BASÉ SUR UNE MESURE DE FLUORESCENCE UTILISANT UNE CARTOUCHE À ÉCOULEMENT LATÉRAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.05.2017   KR 20170058590**
**09.04.2018   KR 20180041225**

(43) Date of publication of application:
**14.11.2018   Bulletin 2018/46**

(73) Proprietor: **Boditech Med Inc.**
**Gangwon-do 24398 (KR)**

(72) Inventors:
• **CHOI, Euiyul**
**Chuncheon-si, Gangwon-do 24214 (KR)**
• **JOO, Hoodon**
**Chuncheon-si, Gangwon-do 24449 (KR)**
• **KIM, Jinsu**
**Chuncheon-si, Gangwon-do 24216 (KR)**

(74) Representative: **Hiebl, Inge Elisabeth**
**Kraus & Weisert**
**Patentanwälte PartGmbB**
**Thomas-Wimmer-Ring 15**
**80539 München (DE)**

(56) References cited:
**WO-A1-2017/087831        WO-A1-2017/127833**
**US-A1- 2014 370 616        US-A1- 2017 292 964**

• **ILESH SHAH MALAY ET AL: "ImageQuant: An image-based quantitative Immunoassay Analyzer", 2017 IEEE INTERNATIONAL SYMPOSIUM ON MEDICAL MEASUREMENTS AND APPLICATIONS (MEMEA), IEEE, 7 May 2017 (2017-05-07), pages 420-425, XP033126296, DOI: 10.1109/MEMEA.2017.7985913 [retrieved on 2017-07-19]**

EP 3 401 681 B1

**Description**

FIELD OF THE INVENTION

The present invention relates to a method for quick quantification assay of 25-hydroxyvitamin D based on fluorescence measurement using lateral flow cartridge.

BACKGROUND OF THE INVENTION

Vitamin D is a fat soluble vitamin useful for forming and maintaining healthy bones according to regulation of calcium and phosphorus levels, and it also plays a role of regulating an immune system and a blood glucose level and suppressing an occurrence of various infections. If there is a deficiency of vitamin D, not only the occurrence rate of osteoporosis, fall, and hip fracture increases but also higher onset rate of various cancers or auto-immune diseases is caused.

Vitamin D (calciferol) is classified into vitamin $D_2$ (ergocalciferol) and vitamin $D_3$ (cholecalciferol). Vitamin $D_2$ is produced from yeast and ergosterol, which is a plant sterol, while vitamin $D_3$ is produced from 7-dehydrocholesterol as a cholesterol precursor when skin is exposed to solar UV radiation. It is known that they have almost the same efficiency.

Vitamin D is present in pro-hormone form as a precursor of active hormone regardless of whether it has been synthesized in a living organism or taken from foods, and it can exhibit the biological activity only after being converted to active form in liver and kidney. In liver, vitamin $D_3$ and vitamin $D_2$ are metabolized to 25-hydroxyvitamin $D_3$ (25-OH-$D_3$) and 25-hydroxyvitamin $D_2$ (25-OH-$D_2$), respectively, as a result of hydroxylation of the carbon at position 25. Those 25-hydroxyvitamin $D_3$ and 25-hydroxyvitamin $D_2$ are transported to kidney, and according to further hydroxylation of the carbon atom at position 1, 25-hydroxyvitamin $D_3$ is metabolized to active hormone 1,25-dihydroxyvitamin $D_3$ and 25-hydroxyvitamin $D_2$ is metabolized to active hormone 1,25-dihydroxyvitamin $D_2$.

The most suitable assay for determining and monitoring the deficiency or hypervitaminosis of vitamin D is to measure 25-hydroxyvitamin D, and for measuring an accurate amount of 25-hydroxyvitamin D, both 25-hydroxyvitamin $D_3$ (25-OH-$D_3$) and 25-hydroxyvitamin $D_2$ (25-OH-$D_2$) need to be measured.

As an assay method for vitamin D that is generally used, there are LC-MS/MS (Liquid Chromatography-Mass Spectrometry/Mass Spectrometry) method and chemiluminescence immunoassay (CLIA). The LC-MS/MS method has an advantage that it has excellent accuracy as a standard assay method for vitamin D, has excellent sensitivity and precision, and allows discriminative measurement of vitamin $D_3$ and $D_2$. However, the LC-MS/MS method has a disadvantage that it requires a rather expensive analytical instrument. On the other hand, the CLIA method has an advantage that quick measurement can be achieved as it uses an automated instrument, has high sensitivity and precision, and does not require any pre-treatment process. However, the CLIA method has a disadvantage that, as there is a huge deviation depending on reagents or specific methods that are employed, the results may vary depending on test authority.

Meanwhile, in Korean Patent Application Publication No. 2015-0100935, "Vitamin D measurement method and measurement kit" characterized by treatment of a sample with a surfactant having a steroid skeleton is disclosed. In addition, in Korean Patent Application Publication No. 2015-0110680, "Method and kit for detecting 1,25-dihydroxyvitamin D and related antibodies" is disclosed. However, the method for quick quantification assay of vitamin D based on fluorescence measurement by using lateral flow cartridge as described in the present invention has never been disclosed.

US 2014/0370616 discloses a method for detecting analytes, particularly vitamins using a lateral flow immunoassay. The method may be used to detect vitamin D, particularly 25-hydroxy vitamin $D_3$ or 1,25-dihydroxy vitamin $D_3$. US2017/292964 discloses a method for detecting vitamin D in a blood sample wherein the sample is combined with metaperiodate and manganese. WO2017/127833 discloses a lateral flow device for detecting vitamin D in a sample. WO/2017087831discloses lateral flow assay strip that allows measurement of vitamin D.

DETAILED DESCRIPTION OF THE INVENTION

TECHNICAL PROBLEMS TO BE SOLVED

The present invention is devised in view of the demand described above, and the inventors of the present invention developed a method for quick quantification of concentration of 25-hydroxyvitamin D present in blood based on immunoassay using anti-25-hydroxyvitamin D antibody, and lateral flow and fluorescence measurement method, and succeeded in optimizing temperature conditions that are required for accurate discrimination of fluorescence values in accordance with a varying amount of 25-hydroxyvitamin D present in sample. The present invention is completed accordingly.

TECHNICAL MEANS FOR SOLVING THE PROBLEMS

To solve the problem described above, the present invention provides a method for quick quantification assay of 25-hydroxyvitamin D based on fluorescence measurement using lateral flow cartridge comprising: (a) preparing a first reaction product by mixing a biological sample with a releasing buffer followed by reaction for 3 to 7 minutes at 32 to 37°C; (b) preparing a second reaction product by adding a detection buffer to the first reaction product of the step (a) followed by reaction for 13 to 17 minutes at 32 to 37°C; and (c) loading the second reaction product of the step (b) in a lateral flow cartridge followed by reaction for 6 to 10 minutes and measuring fluorescence intensity by using a fluorescence meter, wherein the releasing buffer of the step (a) contains 0.4 to 0.6 N sodium hydroxide and 35 to 45% (v/v) DMSO (dimethyl sulfoxide), and wherein the detection buffer of the step (b) contains fluorescent-labeled anti-25-hydroxyvitamin D antibody, and wherein the lateral flow cartridge of the step (c) has test strip form in which a sample injection part to which a sample is injected; a test line placed at a certain distance from the sample injection part, to which a complex of bovine serum albumin and 25-hydroxyvitamin D is immobilized; and a control line to which rabbit IgG is immobilized are provided in order.

ADVANTAGEOUS EFFECT OF THE INVENTION

The present invention relates to a method for quantification of 25-hydroxyvitamin D in a biological sample by using fluorescence measurement and a lateral flow cartridge, and as the method of the present invention does not require a conventional sample pre-treatment step, which is both time consuming and labor intensive, quick and accurate measurement of 25-hydroxyvitamin D can be achieved.

BRIEF DESCRIPTION OF THE DRAWINGS

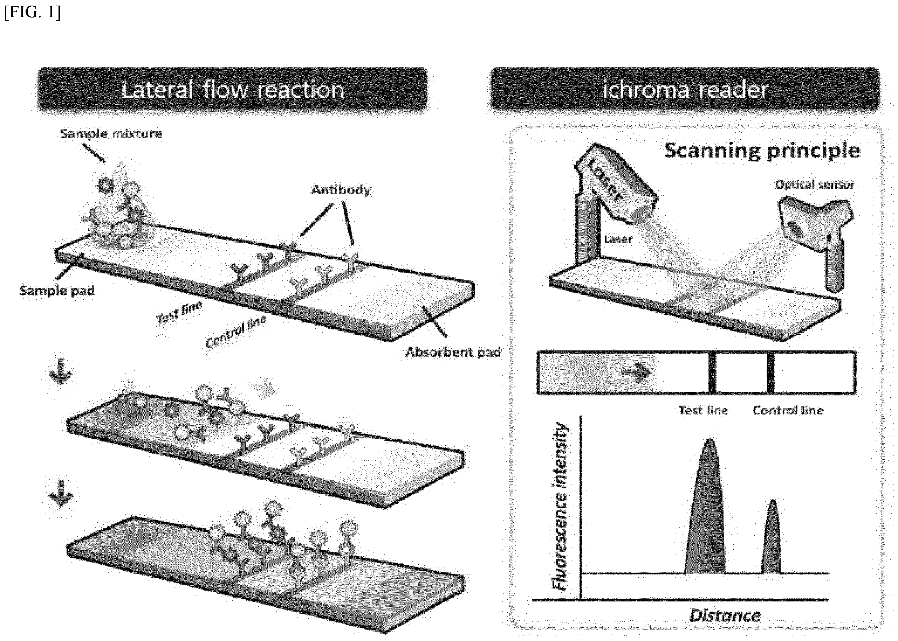

Fig. 1 is a diagrammatical view illustrating the principle of a lateral flow reaction and the principle of fluorescence measurement that are utilized for the vitamin quantification assay method of the present invention.
Fig. 2 is a graph illustrating the analysis of the fluorescence measurement result in accordance with vitamin D concentration at various temperature conditions, in which T area indicates fluorescence value of test line and C area indicates fluorescence value of control line.
Fig. 3 is a graph illustrating the measurement results obtained with the method and instrument of the present invention and the results of quantification analysis obtained with a control (i.e., reference) instrument (Cobas E 411), in which 45 blood serum analytes were used, and Y axis of the graph represents a measurement value of the reference instrument Cobas and X axis of the graph represents a measurement value of the present invention.

BEST MODE(S) FOR CARRYING OUT THE INVENTION

To achieve the object of the present invention, the present invention provides a method for quick quantification assay of 25-hydroxyvitamin D in a biological sample based on fluorescence measurement using lateral flow cartridge.
For the method for quick quantification assay of 25-hydroxyvitamin D of the present invention, an immunoassay utilizing a competitive reaction is used. Specifically, a fluorescent-labeled probe antibody (anti-25-hydroxyvitamin D antibody) present in a detection buffer and vitamin D (25-hydroxyvitamin D; 25(OH)D$_2$ and 25(OH)D$_3$) present in an analyte (i.e., biological sample) form an antigen-antibody complex, a probe antibody left without forming the complex moves along a nitrocellulose medium in a strip of a lateral flow cartridge to compete with a competitor (i.e., BSA-25(OH)D complex) that is immobilized on a nitrocellulose development membrane, and fluorescence emitted from the probe antibody bound to the competitor is measured with a fluorescence meter (ichroma™ Reader, manufactured by Boditech Med Inc.).
The term "immunoassay" means a method for measuring a trace amount of a target material in a sample having large amount of impurities by taking advantage of a specific antigen-antibody binding capability. If the immunoassay is combined with a labeling method which uses a fluorescent (or luminescent) material, a radioactive compound, an enzyme, a metal, or the like, the measurement sensitivity can be dramatically increased.
The method for quick quantification assay of 25-hydroxyvitamin D according to the present invention may comprise:

(a) preparing a first reaction product by mixing a biological sample with a releasing buffer followed by reaction for 3 to 7 minutes at 32 to 37°C;
(b) preparing a second reaction product by adding a detection buffer to the first reaction product of the step (a) followed by reaction for 13 to 17 minutes at 32 to 37°C; and

(c) loading the second reaction product of the step (b) in a lateral flow cartridge followed by reaction for 6 to 10 minutes and measuring fluorescence intensity using a fluorescence meter, wherein the releasing buffer of the step (a) contains 0.4 to 0.6 N sodium hydroxide and 35 to 45% (v/v) DMSO (dimethyl sulfoxide), and wherein the detection buffer of the step (b) contains fluorescent-labeled anti-25-hydroxyvitamin D antibody, and wherein the lateral flow cartridge of the step (c) has test strip form in which a sample injection part to which a sample is injected; a test line placed at a certain distance from the sample injection part, to which a complex of bovine serum albumin and 25-hydroxyvitamin D is immobilized; and a control line to which rabbit igG is immobilized are provided in order.

According to the quick quantification assay method of vitamin D of the present invention, vitamin D is 25-hydroxyvitamin $D_3$ (25-OH-$D_3$) and 25-hydroxyvitamin $D_2$ (25-OH-$D_2$), but it is not limited thereto.

During the last couple of decades, 1,25(OH)D as fully active form of vitamin D is measured for determining the concentration of vitamin D in a living body. However, since 25(OH)D in liver or blood is converted to 1,25(OH)D in kidney, measurement of 1,25(OH)D is presently regarded as a reflection of kidney function instead of vitamin storage amount in a living body. Thus, in recent years, 25(OH)D concentration in blood is measured for determining the vitamin D concentration in a living body.

Furthermore, according to the quick quantification assay method of 25-hydroxyvitamin D of the present invention, the releasing buffer of the step (a) contains 0.4 to 0.6 N sodium hydroxide and 35 to 45% (v/v) DMSO (dimethyl sulfoxide). The releasing buffer may be used for releasing 25-hydroxyvitamin D in a biological sample from a vitamin D binding protein.

As described herein, the term "biological sample" may be a liquid phase or a liquid-like fluid material, for example, blood, saliva, urine, sweat, interstitial fluid, intracellular fluid, or a material extracted from them, and the blood can be whole blood, blood plasma, blood serum, or blood, blood plasma, or blood serum obtained after a certain treatment (for example, anti-coagulation), or the like, but it is not limited thereto. The biological sample may be used either it is in a processed state or it is in a non-processed state.

Furthermore, according to the quick quantification assay method of 25-hydroxyvitamin D of the present invention, the detection buffer of the step (b) contains fluorescent-labeled anti-25-hydroxyvitamin D antibody, and specifically it may consist of fluorescent-labeled anti-25-hydroxyvitamin D antibody, fluorescent-labeled anti-rabbit IgG, 0.3 to 0.7% (w/v) gelatin, 100 to 200 mM sodium chloride, 0.05 to 0.15% (w/v) sodium azide, 0.3 to 0.55% (w/v) CHAPS (3-[(3-cholamidopropyl)dimethylammonio]-1-propanesulfonate hydrate), and 400 to 600 mM Tris-HCl.

Anti-25-hydroxyvitamin D antibody in the detection buffer may bind to both of 25-hydroxyvitamin $D_2$ and 25-hydroxyvitamin $D_3$, and a commercially available antibody can be purchased and used.

Furthermore, according to the quick quantification assay method of 25-hydroxyvitamin D of one embodiment of the present invention, the lateral flow cartridge may be in test strip form in which a sample injection part to which a sample is injected; a test line placed at a certain distance from the sample injection part, to which a complex of bovine serum albumin (BSA) and 25-hydroxyvitamin D is immobilized; and a control line to which rabbit IgG is immobilized are provided in order, but it is not limited thereto.

Furthermore, the quick quantification assay method of 25-hydroxyvitamin D of one embodiment of the present invention is characterized in that the fluorescence intensity is inversely proportional to the concentration of vitamin D in a biological sample, and that is because, among fluorescent-labeled anti-25-hydroxyvitamin D antibodies present in detection buffer, the anti-25-hydroxyvitamin D antibody in free form which does not form an antigen-antibody complex is captured by BSA-25-hydroxyvitamin D complex immobilized at the test line, and the fluorescence amount emitted therefrom is measured. Namely, if 25-hydroxyvitamin D is present in a large amount in blood, most of anti-25-hydroxyvitamin D antibody form an antigen-antibody complex, and thus the amount of anti-25-hydroxyvitamin D antibody in free form would be lower. Accordingly, as the number of anti-25-hydroxyvitamin D antibody to be bound to BSA-25-hydroxyvitamin D immobilized at the test line of a lateral flow cartridge is small, a final fluorescence signal with weak intensity will be shown. On the other hand, if 25-hydroxyvitamin D is present in a small amount in blood, a final fluorescence signal with strong intensity will be shown.

Another fluorescent-labeled anti-rabbit IgG in the detection buffer is employed as an internal control, and according to binding to rabbit IgG immobilized at the control line of a lateral flow cartridge, fluorescence is exhibited. As such, as long as it is a normal reaction, a constant fluorescence amount will be shown from every reaction.

In the present invention, a fluorescent material is used as a label to detect an antigen-antibody complex, but it is not limited thereto. An enzyme, a ligand, a light-emitting material, a microparticle, or a radioactive isotope, or the like can be also used.

Examples of the fluorescent material used as a detection label include fluorescein, isothiocyanate, rhodamine, phycoerythrin, phycocyanin, allophycocyanin, o-phthaldialdehyde, fluorescamine, and $Eu^{3+}$, $Eu^{3+}$ chelate or cryptate. Examples of the enzyme include acetylcholine esterase, alkaline phosphatase, $\beta$-D-galactosidase, horseradish peroxidase, and $\beta$-lactamase. Examples of the ligand include biotin derivatives or the like. Examples of the light-emitting material include acridinium ester, isoluminol derivatives, or the like. Examples of the microparticle include colloidal gold, colored latex, or the like. Examples of the radioactive isotope include $^{57}$Co, $^{3}$H, $^{125}$I, $^{125}$I-Bolton Hunter reagent, or the like, but it is not limited thereto.

The range of 25-hydroxyvitamin D concentration in a biological sample that can be quantitatively assayed by the method of the present invention is 8.0 to 70 ng/mL.

Hereinbelow, the present invention is explained in greater detail in view of the Examples. However, it is evident that the following Examples are given only for exemplification of the present invention and by no means the present invention is limited to the following Examples.

Materials and Methods

1. Reagents and instruments

Fluorescent-labeled anti-25(OH)D antibody and anti-rabbit IgG antibody were prepared by separately purchasing each of anti-25(OH)D antibody, anti-rabbit IgG antibody, and FPR-648 followed by conjugation. Bovine serum albumin and 25-hydroxyvitamin $D_3$ (25(OH)$D_3$), which are to be immobilized at the test line, were also prepared by separate purchase and conjugation of them.

The releasing buffer was prepared to have composition of 0.5 N sodium hydroxide and 40% DMSO, while the detection buffer was prepared to have composition of 0.5% gelatin, 150 mM sodium chloride, 0.1% sodium azide (NaN$_3$), 0.4% CHAPS, 500 mM Tris-HCl (pH 6.8), fluorescent-labeled anti-25(OH)D antibody, and fluorescent-labeled anti-rabbit IgG.

2. Calculation of vitamin D concentration based on fluorescent value

The fluorescent amount measured at the test line (i.e., T area) was divided with the fluorescent amount measured at the control line (i.e., C area), and the resulting value was designated as "ratio."

Calibrators with 8 different concentrations (0, 5, 10, 20, 30, 50, 70, and 100 ng/ml), which have been previously measured by using a standard instrument, were prepared, and the ratio value at each concentration condition was determined according to measurement with a fluorescence meter (ichroma™ Reader, manufactured by Boditech Med Inc.). Based on those ratio values, a calibration curve was established. By incorporating a ratio obtained from measurement of an unknown sample to the calibration curve, concentration of vitamin D in sample is calculated.

Example 1. Analysis of change in result depending on temperature conditions

With regard to the measurement of the concentration of vitamin D in sample, the influence of temperature of the reaction condition on the concentration measurement was evaluated. Specifically, after mixing 50µℓ of the releasing buffer with 50µℓ of a vitamin D sample with concentration of 0, 10, 30, or 70 ng/ml, the reaction was allowed to proceed for 5 minutes at temperature conditions of 20, 22, 25, 30, 32, 35, 37, or 39°C. After that, 100µℓ of the detection buffer was added to the reaction product, and the reaction was allowed to proceed for 15 minutes at the above 8 different temperature conditions. To the sample injection part of a test cartridge placed on a slot of a fluorescence meter, the reaction product was loaded in an amount of 75 µℓ, and 8 minutes after closing the slot of a fluorescence meter, the fluorescence intensity was measured from the control line and the test line, respectively.

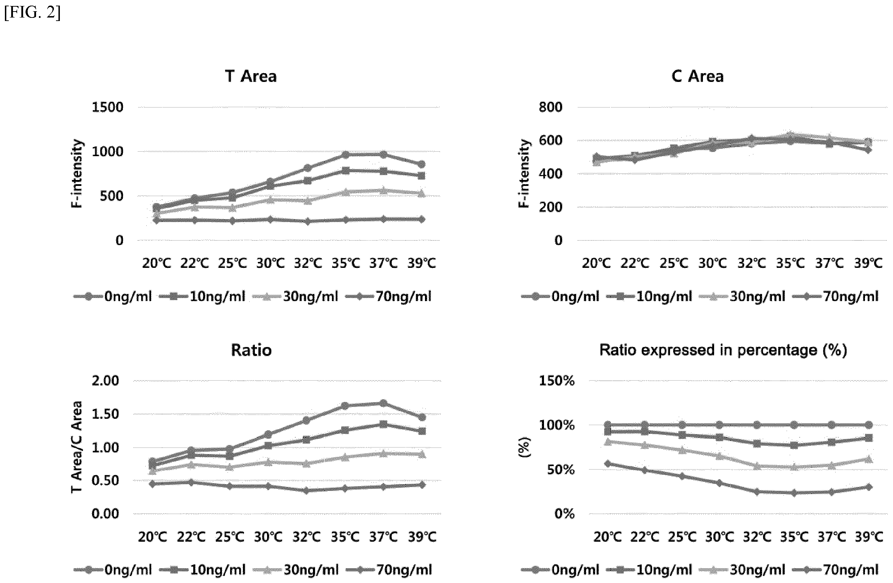

As a result, the fluorescence amount from control line (i.e., C area) shows a constant value in all cases as it is shown in Fig. 2, regardless of sample concentration and temperature conditions. However, at temperature conditions of 20, 22, 25, and 30°C, it was found that the fluorescence amount from the test line (i.e., T area) is not distinguished between vitamin D of zero (0) concentration and vitamin D of 10 ng/ml concentration. Furthermore, at 39°C, there is a tendency that the difference in fluorescence measurement value among different concentrations decreases again compared to the temperature conditions of 32, 35, and 37°C. According to the examination of a ratio obtained by dividing the fluorescent amount measured at the test line (i.e., T area) by the fluorescent amount measured at the control line (i.e., C area) and the same ratio expressed in percentage, a significant difference in fluorescence measurement value was shown in accordance with the concentration of a vitamin D sample, and thus the temperature conditions that are more advantageous for discrimination of sample concentration were found to be within a range of from 32 to 37°C.

Example 2. Performance analysis using standard condition

Based on the above Example 1, the method of the present invention was set to perform at 35°C temperature condition, and the precision and accuracy were analyzed for the sample analysis using the method of the present invention.

For the precision determination, the measurement results for reference materials were analyzed for each of production lot (i.e., between-lot), tester (i.e., between-person), repetition number for same lot/day (i.e., between-day), and performing site of same lot (i.e., between-site).

Table 1

| Analysis of precision | | | | | | | | |
|---|---|---|---|---|---|---|---|---|
| Concentration of reference material (ng/ml) | Between-lot | | Between-person | | Between-day | | Between-site | |
| | Mean | Variation coefficient* | Mean | Variation coefficient | Mean | Variation coefficient | Mean | Variation coefficient |
| 9.59 | 9.63 | 9.12 | 9.37 | 8.80 | 9.40 | 11.26 | 9.53 | 10.03 |
| 23.76 | 23.08 | 5.87 | 23.03 | 5.87 | 24.26 | 6.11 | 23.02 | 6.68 |
| 64.78 | 64.29 | 5.30 | 64.21 | 4.65 | 64.58 | 4.25 | 64.64 | 3.56 |
| * Variation coefficient: Percentage (%) of standard deviation compared to mean | | | | | | | | |

As a result, it was able to confirm that there is not much difference in the measurement results depending on between-lot, between-person, between-day, or between-site at various conditions as shown in Table 1. Furthermore, as a result of measuring repeatedly 10 times the reference materials with 3 different concentrations and 3 different lots, the recovery rate was found to be at the level of 97 to 100% as shown in Table 2, indicating the measurement of the same value as the reference material concentration. Based on that, it was recognized that method for measuring vitamin D of the present invention has excellent accuracy.

Table 2

| Analysis of accuracy | | | | | | | |
|---|---|---|---|---|---|---|---|
| Concentration of reference material (ng/ml) | Lot 1 | Lot 2 | Lot 3 | Mean | Standard deviation | Variation coefficient (%) | Recovery rate*(%) |
| 9.59 | 9.56 | 9.74 | 9.61 | 9.63 | 0.88 | 9.12 | 100.46 |
| 23.76 | 22.71 | 22.84 | 23.68 | 23.08 | 1.35 | 5.87 | 97.13 |
| 64.78 | 63.21 | 65.05 | 64.62 | 64.29 | 3.41 | 5.30 | 99.25 |
| *Recovery rate: Mean of measurement values/Reference material concentration x 100 | | | | | | | |

Example 3. Comparative correlationship

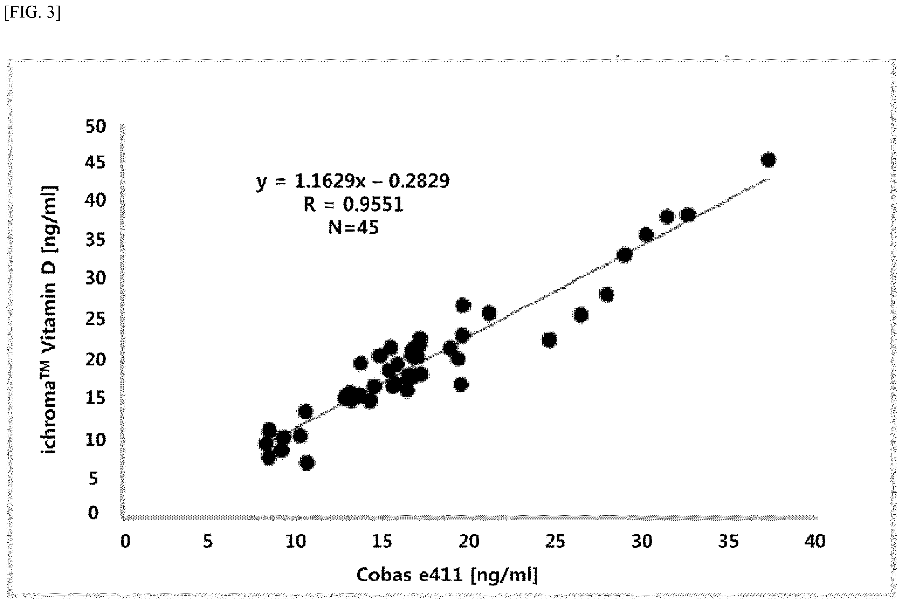

45 Blood serum analytes were used to determine vitamin D concentration by using the fluorescence measurement instrument of the present invention or Cobas E 411 (Roche, Switzerland) as an immunochemical analyzer, followed by comparative analysis. As a result, the quantitative analysis results showed a linear regression equation of $Y = 1.1629X - 0.2829$ as shown in Fig. 3, while showing the correlation coefficient (R) of 0.9551. Based on those results, it was found that the measurement values of the present invention are not much different from measurement values that are obtained by using a standard instrument.

**Claims**

1. A method for quick quantification assay of 25-hydroxyvitamin D based on fluorescence measurement using lateral flow cartridge comprising:

   (a) preparing a first reaction product by mixing a biological sample with a releasing buffer followed by reaction for 3 to 7 minutes at 32 to 37°C;
   (b) preparing a second reaction product by adding a detection buffer to the first reaction product of the step (a) followed by reaction for 13 to 17 minutes at 32 to 37°C; and
   (c) loading the second reaction product of the step (b) in a lateral flow cartridge followed by reaction for 6 to 10 minutes and measuring fluorescence intensity using a fluorescence meter, wherein the releasing buffer of the step (a) contains 0.4 to 0.6 N sodium hydroxide and 35 to 45% (v/v) DMSO (dimethyl sulfoxide), and wherein the detection buffer of the step (b) contains fluorescent-labeled anti-25-hydroxyvitamin D antibody, and wherein the lateral flow cartridge of the step (c) has test strip form in which a sample injection part to which a sample is injected; a test line placed at a certain distance from the sample injection part, to which a complex of bovine serum albumin and 25-hydroxyvitamin D is immobilized; and a control line to which rabbit IgG is immobilized are provided in order.

**Patentansprüche**

1. Verfahren zur schnellen quantitativen Bestimmung von 25-Hydroxyvitamin D auf der Basis von Fluoreszenzmessung unter Verwendung einer Lateral-Flow-Kartusche, umfassend:

   (a) Herstellung eines ersten Reaktionsprodukts durch Mischen einer biologischen Probe mit einem Freisetzungspuffer, gefolgt von einer Reaktion für 3 bis 7 Minuten bei 32 bis 37 °C;
   (b) Herstellung eines zweiten Reaktionsprodukts durch Zugabe eines Nachweispuffers zum ersten Reaktionsprodukt aus Schritt (a), gefolgt von einer Reaktion für 13 bis 17 Minuten bei 32 bis 37°C; und
   (c) Laden des zweiten Reaktionsprodukts aus Schritt (b) in eine Lateral-Flow-Kartusche, gefolgt von einer Reaktion für 6 bis 10 Minuten und Messen der Fluoreszenzintensität unter Verwendung eines Fluoreszenzmessgeräts, wobei der Freisetzungspuffer aus Schritt (a) 0,4 bis 0,6 N Natriumhydroxid und 35 bis 45% (V/V) DMSO (Dimethylsulfoxid) enthält, und wobei der Nachweispuffer aus Schritt (b) einen fluoreszenzmarkierten Anti-25-Hydroxyvitamin-D-Antikörper enthält, und wobei die Lateral-Flow-Kartusche aus Schritt (c) die Form eines Teststreifens aufweist, in dem ein Probeninjektionsteil, in den eine Probe injiziert wird; eine Testlinie, die in einem bestimmten Abstand von dem Probeninjektionsteil angeordnet ist, an der ein Komplex aus Rinderserumalbumin und 25-Hydroxyvitamin D immobilisiert ist; und eine Kontrolllinie, an der Kaninchen-IgG immobilisiert ist, in dieser Reihenfolge bereitgestellt sind.

**Revendications**

1. Procédé de dosage de quantification rapide de la 25-hydroxyvitamine D sur la base d'une mesure de fluorescence à l'aide d'une cartouche à écoulement latéral comprenant :

   (a) la préparation d'un premier produit de réaction en mélangeant un échantillon biologique avec un tampon de libération suivi d'une réaction pendant 3 à 7 minutes à 32 à 37 °C ;
   (b) la préparation d'un second produit de réaction en ajoutant un tampon de détection au premier produit de réaction de l'étape (a) suivi d'une réaction pendant 13 à 17 minutes à 32 à 37 °C ; et

(c) le chargement du second produit de réaction de l'étape (b) dans une cartouche à écoulement latéral suivi d'une réaction pendant 6 à 10 minutes et la mesure de l'intensité de fluorescence à l'aide d'un appareil de mesure à fluorescence, le tampon de libération de l'étape (a) contenant 0,4 à 0,6 N d'hydroxyde de sodium et 35 à 45 % (v/v) de DMSO (diméthylsulfoxyde), et le tampon de détection de l'étape (b) contenant un anticorps anti-25-hydroxyvitamine D marqué par fluorescence, et la cartouche à écoulement latéral de l'étape (c) se présentant sous la forme d'une bande de test dans laquelle une partie d'injection d'échantillon dans laquelle un échantillon est injecté ; une ligne d'essai placée à une certaine distance de la partie d'injection d'échantillon, sur laquelle un complexe d'albumine sérique bovine et de 25-hydroxyvitamine D est immobilisé ; et une ligne de commande vers laquelle l'IgG de lapin est immobilisé, sont fournis dans l'ordre.

[FIG. 1]

**Lateral flow reaction**

Sample mixture

Antibody

Sample pad

Test line

Control line

Absorbent pad

**ichroma reader**

**Scanning principle**

Laser

Optical sensor

Laser

Test line    Control line

Fluorescence intensity

Distance

EP 3 401 681 B1

[FIG. 2]

[FIG. 3]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20150100935
- KR 20150110680
- US 20140370616 A
- US 2017292964 A
- WO 2017127833 A
- WO 2017087831 A